# EUROPEAN PATENT APPLICATION

(11) **EP 4 361 636 A1**
(43) Date of publication of application: **01.05.2024**
(21) Application number: 23204019.6
(22) Date of filing: 17.10.2023
(51) Int. Cl.: G01N 33/68, H01J 49/04, C07H 21/02

(54) **ANALYZING METHOD FOR RNA**

(30) Priority: 27.10.2022 JP 2022172722
(71) Applicant: Shimadzu Corporation, Nakagyo-ku, Kyoto-shi, Kyoto 604-8511 (JP)
(72) Inventor: FUKUYAMA, Yuko, Nakagyo-ku, Kyoto-shi, 604-8511 (JP)
(74) Representative: Müller-Boré & Partner Patentanwälte PartG mbB

(57) **Abstract**

[OBJECT] To enable a more sensitive and uniform detection of a molecular-related ion in an analysis of an RNA contained in a sample by means of MALDI mass spectrometry. [MEANS FOR SOLVING PROBLEM] A mixed matrix containing 2,4-dihydroxyacetophenone (DHAP) and 2,4,6-trihydroxyacetophenone monohydrate (TRAP) is used as a matrix to analyze an RNA contained in a sample by a matrix-assisted laser desorption/ionization mass spectrometer.

## Description

### TECHNICAL FIELD

The present invention relates to a method for analyzing an RNA.

### BACKGROUND ART

A mass spectrometry method employing matrix assisted laser desorption/ionization (MALDI; this method is hereinafter called the MALDI mass spectrometry) has been known as one of the methods for mass spectrometric analyses of nucleic acids (DNA and RNA). In the MALDI mass spectrometry, an ionization assistant reagent called the matrix is used for ionizing a substance that barely absorbs laser light or a substance that easily undergoes damage due to laser light.

For example, in the MALDI mass spectrometry for nucleic acids, Non Patent Literature 1 describes that 3-hydroxypicolinic acid (3-HPA) and 1,5-diaminonaphthalene (1,5-DAN) are used as matrices. Non Patent Literature 2 describes that 2,4-dihydroxyacetophenone (2,4-DHAP) is used as a matrix. Non Patent Literature 3 describes that a mixed matrix containing 2,4,6-trihydroxyacetophenone (2,4,6-THAP) and 2,3,4-trihydroxyacetophenone (2,3,4-THAP), as well as a mixed matrix containing anthranilic acid (AA) and nicotinic acid (NA), are used as matrices.

A matrix is normally prepared as a matrix solution in which the matrix is dissolved in a predetermined solvent, and this solution is to be mixed, by various methods, with a sample containing an analysis-target substance. For example, a matrix solution and a sample solution are previously mixed together to prepare a sample/matrix mixture solution. This sample/matrix mixture solution is dropped onto one of the wells formed on a metallic plate called a "sample plate" (the wells are sample-receiving portions, each of which is, for example, a circle of approximately 2 mm in diameter with its outer edge grounded, or a circle of approximately 2 mm in diameter with the surface of its inner area entirely treated), and is dried to form a sample/matrix mixture crystal consisting of the analysis-target substance and the matrix mixed together on the well. Alternatively, the matrix solution and the sample solution are separately dropped onto a well, mixed on the same well, and dried to form a sample/matrix mixture crystal. In any of these cases, the sample/matrix mixture crystal is subjected to a MALDI mass spectrometric analysis as an analysis sample.

### CITATION LIST

### NON PATENT LITERATURE

Non Patent Literature 1: Nathan A. Hagan and five other authors, "Enhanced In-Source Fragmentation in MALDI-TOF-MS of Oligonucleotides Using 1,5-Diaminonapthalene", Journal of the American Society for Mass Spectrometry, (USA), 2012, 23, pp.773-777

Non Patent Literature 2: H. Shimizu and six other authors, "Application of high-resolution ESI and MALDI mass spectrometry to metabolite profiling of small interfering RNA duplex", Journal of Mass Spectrometry, (USA), 2012, 47, pp. 1015-1022

Non Patent Literature 3: Li-Kang Zhang and another author, "Matrix-assisted laser desorption/ionization mass spectrometry methods for oligodeoxynucleotides: Improvements in matrix, detection limits, quantification, and sequencing", Journal of the American Society for Mass Spectrometry, (USA), 2000, 11, pp. 854-865

### SUMMARY OF INVENTION

### TECHNICAL PROBLEM

Conventionally, when an analysis sample is prepared by using a matrix commonly known as a matrix for MALDI mass spectrometry of nucleic acids, it is often the case that the sample/matrix mixture crystal is non-uniformly formed on the well. Therefore, when a beam of laser light is delivered onto the well to obtain a "molecular-related ion" ([M+H]⁺ or [M-H]⁻, where M is a molecule and H is a hydrogen atom) of a nucleic acid which is the analysis-target substance, it may be impossible to produce a sufficient amount of molecular-related ion at some of the irradiation positions. A satisfactory peak of the molecular-related ion can still be detected with a sufficient level of sensitivity when the laser light is successfully delivered onto a site of the well where the sample/matrix mixture crystal is formed and where the ion of the sample molecule as the analysis-target substance is relatively easy to be detected (such a site is called a "sweet spot"). However, for an unskilled measurement operator, it is difficult to quickly locate such a site, and the analysis requires a considerable amount of time.

A raster scan function can be used to perform the laser irradiation on a sample/matrix mixture crystal formed on a well, whereby the mass spectrometric analysis of the analysis-target substance contained in the sample/matrix mixture crystal can be performed more easily and speedily. However, when the sample/matrix mixture crystal is non-uniformly formed on the well, causing the crystal site on the well to be uneven in size, or when the sample/matrix mixture crystal is non-uniform and the distribution (location) of the sample within the mixture crystal is considerably uneven, the amount of ion generated from the analysis-target substance tends to vary depending on the irradiation position, which causes, for example, a situation in which the molecular-related ion of the analysis-target substance is excessively produced, or conversely, a sufficient amount of molecular-related ion cannot be produced, depending on the irradiation position.

Furthermore, as compared to DNA, RNA has fewer kinds of matrices reported to be available for MALDI mass spectrometry. Additionally, the previously described problems are more likely to occur with the conventional matrices reported for RNA analyses. Thus, a technique which enables a sensitive and rapid analysis of RNAs has been demanded.

The problem for the present invention is to provide a technique which enables a sensitive, easy, and rapid detection of a molecular-related ion in an analysis of an RNA by means of MALDI mass spectrometry.

### SOLUTION TO PROBLEM

A method for analyzing an RNA according to the present invention developed for solving the previously described problem is a method in which a mixed matrix containing 2,4-dihydroxyacetophenone (DHAP) and 2,4,6-trihydroxyacetophenone monohydrate (THAP) is used as a matrix to analyze an RNA contained in a sample by a matrix-assisted laser desorption/ionization mass spectrometer.

A matrix for matrix-assisted laser desorption/ionization mass spectrometry for RNA according to the present invention developed for solving the previously described problem is a matrix containing 2,4-dihydroxyacetophenone (DHAP) and 2,4,6-trihydroxyacetophenone monohydrate (THAP).

### ADVANTAGEOUS EFFECTS OF INVENTION

By the method for analyzing an RNA according to the present invention, when an RNA contained in a sample is analyzed by means of a matrix-assisted laser desorption/ionization mass spectrometer, a comparatively uniform sample/matrix mixture crystal can be created, so that a molecular-related ion can be detected sensitively, easily, and speedily. By preparing an analysis sample of an RNA-containing sample using the matrix for matrix-assisted laser desorption/ionization mass spectrometry for RNA according to the present invention, a comparatively uniform sample/matrix mixture crystal can be created for performing a matrix-assisted laser desorption/ionization mass spectrometric analysis, so that a molecular-related ion can be detected sensitively, easily, and speedily. Therefore, the RNA in the sample can be satisfactorily analyzed.

### BRIEF DESCRIPTION OF DRAWINGS

[Fig. 1] Fig. 1 shows mass spectra of patisiran (sense strand) and a table showing the state of the detection of the molecular-related ion, in the case where various kinds of matrices were used in the first example.
[Fig. 2] Fig. 2 shows mass spectra of patisiran (sense strand) and a table showing the state of the detection of the molecular-related ion, in the case where various kinds of matrices were used in the second example.
[Fig. 3] Fig. 3 shows mass spectra of patisiran (antisense strand) and a table showing the state of the detection of the molecular-related ion, in the case where various kinds of matrices were used in the third example.
[Fig. 4] Fig. 4 shows mass spectra of patisiran (sense strand) and a table showing the state of the detection of the molecular-related ion, in the case where various kinds of matrices were used in the fourth example.
[Fig. 5] Fig. 5 shows mass spectra of mipomersen and a table showing the state of the detection of the molecular-related ion, in the case where various kinds of matrices were used in the first reference example.

### DESCRIPTION OF EMBODIMENTS

Hereinafter, one embodiment of the method for analyzing an RNA according to the present invention is described.

### (Analysis-Target Substance)

RNAs which are analysis-target substances in the present embodiment may be natural substances obtained from living organisms or processed products of those substances, or alternatively, they may be artificial synthetic nucleic acids obtained by chemical synthesis. RNAs include RNA-related substances, such as modified RNAs, RNA derivatives, and RNA drugs. There is no specific limitation on the degree of polymerization (base length) of the RNAs, although oligonucleotides with several to tens of nucleotides polymerized are preferable. Since the analysis method according to the present embodiment particularly enables a sensitive analysis of high-molecular RNAs, the molecular weights of the RNAs may preferably be equal to or greater than 3000, and particularly, equal to or greater than 6000.

### (Preparation of Analysis sample)

An analysis sample is prepared by mixing a sample containing an RNA which is an analysis-target substance, with 2,4-dihydroxyacetophenone (hereinafter denoted as DHAP) and 2,4,6-trihydroxyacetophenone monohydrate (hereinafter denoted as THAP) as the matrix. Specifically, for example, a sample solution and a mixed matrix solution containing DHAP and THAP are prepared and mixed together beforehand to prepare a sample/mixed-matrix mixture solution, or alternatively, a sample solution, a matrix solution containing DHAP, and a matrix solution containing THAP are prepared and mixed together beforehand to prepare a sample/mixed-matrix mixture solution, and the sample/mixed-matrix mixture solution is dropped onto a well and dried (this method is called the "pre-mix method"). The sample solution and the mixed matrix solution may also be separately dropped onto a well, mixed together on the well and dried, or alternatively, the sample solution, the matrix solution containing DHAP, and the matrix solution containing THAP may be separately dropped onto a well, mixed together on the well and dried (this method is called the "on-target method"). Thus, a sample/mixed-matrix mixture crystal which is a mixture crystal of the analysis-target substance and the mixed matrix (and corresponds to the sample/mixed-matrix mixture) is formed on the well.

The mixture ratio of the DHAP and THAP in the mixed matrix (or in the mixed matrix solution) should preferably be such that DHAP:THAP falls within a range from 30:1 to 10:1, and more preferably, from 30:1 to 15:1, since this improves the homogeneity of the mixture crystal as well as enables a sensitive analysis of RNAs.

The matrix, or the matrix solution, may additionally contain diammonium hydrogen citrate as a matrix additive. There are several kinds of ammonium salts of citric acid depending on the number of ammonium ions binding to a citrate ion, of which a salt with two ammonium ions binding to one citrate ion is preferable for use in the present embodiment. The concentration of the diammonium hydrogen citrate in the matrix solution should preferably be within a range from 20 to 100 mM since this enables a sensitive analysis of the molecular-related ion of the RNA. There is no specific limitation on the solvent of the matrix solution; any solvent commonly used as a solvent of a matrix solution may be used. For example, an aqueous solution containing 20-80% organic solvent, such as acetonitrile, methanol, or ethanol, can be used. Among other things, an aqueous solution containing 50-70% acetonitrile is preferable to use, and more specifically, an aqueous solution containing 50% acetonitrile is particularly preferable to use. The matrix additive may be added to a matrix solution containing DHAP or THAP, or it may also be added to a mixed matrix solution containing DHAP and THAP.

### (Mass Spectrometry)

The analysis method according to the present embodiment uses a mass spectrometer having an ion source employing a MALDI method (MALDI-MS). Examples of the MALDI-MS include a MALDI-TOFMS, which is a time of flight (TOF) type of device, and a MALDI-ITMS, which is an ion trap (IT) type of device. The "ion trap type" in the present context means a type of MALDI-ITMS having an ion trap for capturing ions, including a mass spectrometer in which ions captured within the ion trap are sequentially ejected in ascending order of their mass-to-charge ratios (m/z) by a mass-separating function of the ion trap itself and are detected by a detector located outside the ion trap, as well as a mass spectrometer in which ions simultaneously ejected from the ion trap are separated from each other according to their mass-to-charge ratios by a mass separator, such as a time-of-flight mass separator, located outside the ion trap and are detected by a detector which is also located outside the ion trap.

In the case where the ion trap of the MALDI-ITMS is an RF trap which captures and ejects ions by means of a radiofrequency (RF) electric field, it may be an ion trap configured to capture ions by means of an electric field generated by applying a sinusoidal RF voltage to a ring electrode, or a digital ion trap configured to capture ions by means of an electric field generated by applying, to a ring electrode, a rectangular voltage produced by switching between two different voltages at high speeds. In the digital ion trap, the m/z range of the ions that can be captured is controlled by changing the frequency of the rectangular voltage while constantly maintaining its amplitude (voltage value). Scanning a frequency range toward the lower-frequency end causes the trapped ions to be sequentially ejected in ascending order of their m/z. By detecting those ions with a detector, a mass spectrum can be acquired. It is preferable to use a digital ion trap type of MALDI ITMS as the mass spectrometer.

The mass spectrometer may be provided with the function of a measurement by a raster scan. A measurement by a raster scan is a measurement technique in which a set of measurement data is obtained by performing the laser-light irradiation a specified number of times at each of the many measurement points previously set at different positions on a well, and all sets of measurement data are accumulated to derive an ultimate set of measurement data. Since the laser irradiation position is mechanically shifted, the influence of the variation in measurement result due to a difference in the laser irradiation position is reduced in the accumulated data. Since the measurement by a raster scan does not require manually changing the irradiation position, an easy and quick measurement is possible. Additionally, since the measurement by a raster scan is an automatic measurement function with no human intervention for the selection of measurement positions, a more objective and reproducible data acquisition is possible.

There is no specific limitation on the setting conditions of the raster scan; they may be appropriately tuned according to the well diameter of the sample plate, diameter of the laser beam and other parameters of the used device. The laser irradiation should be performed a sufficient number of times for the peak detection so as to maximally avoid an overlap of the laser irradiation positions on the well of the sample plate as well as on the sample/mixed-matrix mixture crystal on the well. For example, for a circular well having a diameter of 2 mm, the set values of the setting conditions of the raster scan may specifically be as follows: 25 points per one well (which indicates that the number of laser irradiation positions on the wells is 25 per one well); 4 shots/point (which indicates that the laser irradiation is performed four times per one laser irradiation position); spacing, 0.2 mm (which indicates that the spatial interval of the laser irradiation positions is 0.2 mm); and size, 0.8 mm (which indicates that the scan direction is turned every time the horizontal scan length reaches 0.8 mm during the laser irradiation of the 25 aforementioned points, or in other words, the value shows the length of the horizontal axis within a square type of laser irradiation area). A square area with 5×5 = 25 points automatically calculated from those settings is subjected to the raster scan.

Hereinafter, a description of the method for analyzing an RNA according to the present invention is given by means of specific examples. These examples are merely illustrative, and the present invention should not be limited to them.

### FIRST EXAMPLE

### <1. Preparation of Sample Solution>

As a sample solution, a 20-pmol/µL aqueous solution of patisiran (5'-G-Um-A-A-Cm-Cm-A-A-G-A-G-Um-A-Um-Um-Cm-Cm-A-Um-dT-dT-3', where dT represents thymidine deoxyribonucleotide, Cm represents 2'-O-methylcytidine, and Um represents 2'-O-methyluridine; Sequence Number 1) was prepared. (This sample was obtained through the desalting purification of a synthetic nucleic acid for research and development. Although patisiran is originally a double-stranded RNA consisting of the sense and antisense strands, only the sense strand was used in the present example. The sense strand had the entire length of 21 bases and a core sequence of 19 bases, with a DNA overhang of two bases (dTdT) at the 3' terminal; MW 6764).

### <2. Preparation of Matrix Solutions>

A 40mg/mL-50% acetonitrile-water solution of 2,4-dihydroxyacetophenone (DHAP) containing 70 mM of diammonium hydrogen citrate as a matrix additive (DHAP solution), and a 40mg/mL-50% acetonitrile-water solution of 2,4,6-trihydroxyacetophenone monohydrate (THAP) containing 70 mM of diammonium hydrogen citrate as a matrix additive (THAP solution), were prepared as matrix solutions. The prepared DHAP and THAP solutions were mixed together at each of the mixture ratios of 30:1, 20:1, 15:1, 10:1, 5:1, 3:1, 1:1, 1:3, 1:5 and 1:10 (v/v) to prepare mixed matrix solutions.

### <3. Preparation of Analysis Sample>

The sample solution prepared in Step 1 and the matrix solution prepared in Step 2 were mixed at 1:1 (v/v), and 1 µL of the obtained mixed solution was dropped onto a well of a sample plate (stainless steel plate). After the solutions had been dropped, the sample plate was placed in an ultra-low humidity drying box (product name: McDry, manufactured by ERC Co., Ltd.) and dried.

### <4. Mass Spectrometric Analysis>

For the mass spectrometric analysis, a MALDI digital ion trap mass spectrometer (MALDI-DITMS; product name: MALDImini-1, manufactured by Shimadzu Corporation) was used. The sample plate in Step 3 was set in the MALDI-DITMS, and a measurement was performed in the positive mode, using the raster scan function. The setting conditions of the raster scan were 4 shots/point and 25 points. As for the laser power, the optimum value for each matrix was used. The laser power depends on the matrix; THAP requires a higher amount of laser power for the peak detection than DHAP. The optimum value of the laser power for each matrix was set by monitoring the state of the peak detection. Specifically, a condition which yielded comparatively high sensitivity and resolution was searched for. It was the mixed matrix that had the lowest optimum value of laser power.

### <Result>

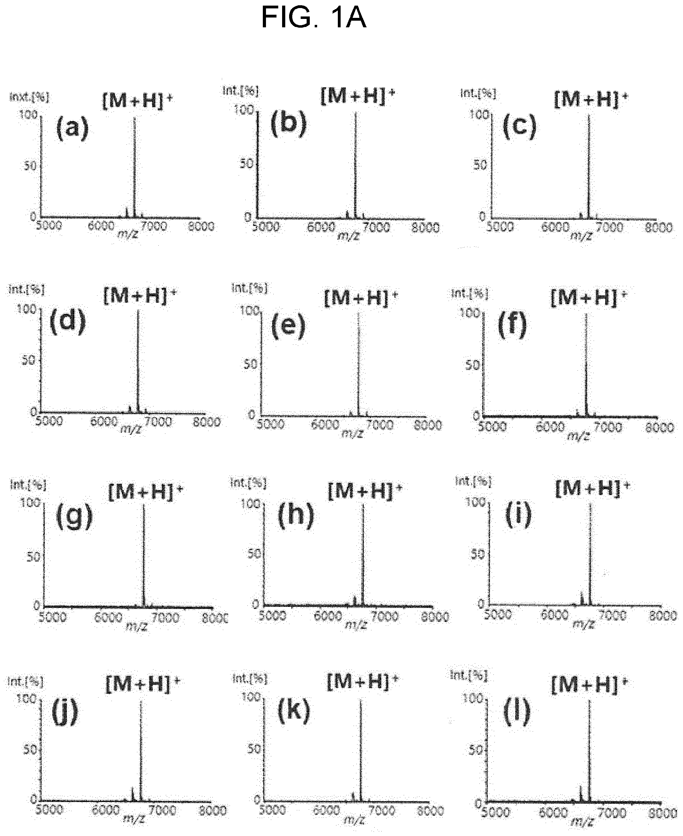

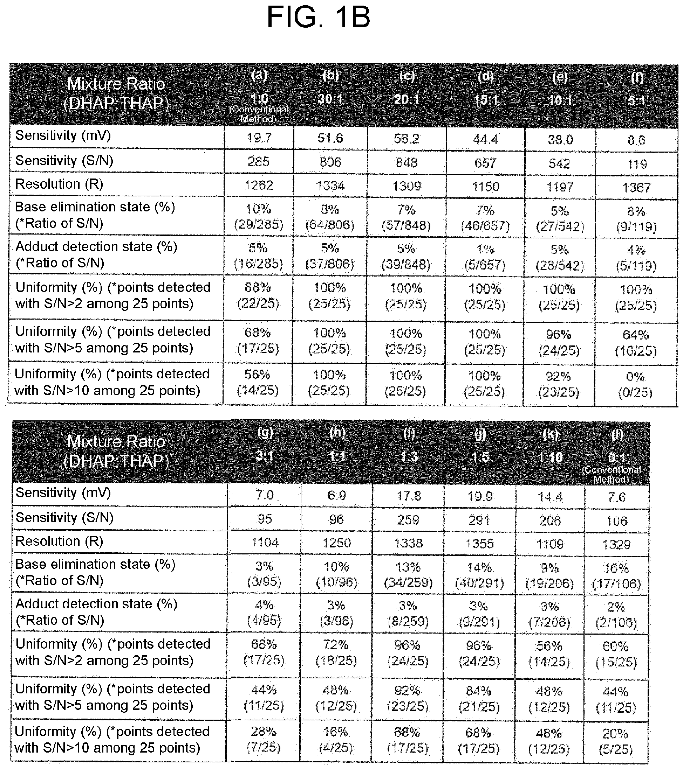

Fig. 1 shows mass spectra of the sense strand of patisiran as well as a table showing the state of the [M+H]⁺ peak detection (sensitivity, resolution, base elimination state, adduct detection state, and uniformity of the peak detection by the raster scan measurement) in the case where DHAP, THAP and mixed matrices (DHAP+THAP) which contained DHAP and THAP at their respective mixture ratios were used as matrices. A mixture ratio of 1:0 shows the case where DHAP was solely used, while a mixture ratio of 0:1 shows the case where THAP was solely used.

The base loss in the base elimination state refers to a phenomenon in which a base is lost from the molecular-related ion ([M+H⁺]) in the ionization process. The ease of occurrence of this phenomenon varies depending on the characteristics of each matrix. For example, a matrix which causes soft ionization is unlikely to cause base loss. On the other hand, a matrix which causes hard ionization is likely to cause base loss.

As for the adduct in the adduct detection state, no detailed analysis was performed. Inferred from the m/z values, the adducts may include oxidized forms (e.g., [M+O]⁺), alkali metal ion adducts (e.g., [M+Na]⁺, [M+O+Na]⁺ and [M+2Na]⁺), base adducts (e.g., [M+B+H]⁺) and matrix ion adducts (e.g., [M+m+H]⁺). An adduct which is not originally present in the sample and yet is produced due to an influence of some kind of foreign substance may have been produced during the process of preparation or ionization, with the ease of its production varying depending on the characteristics of each matrix. The affinity of the matrix for the sample, alkali metal ion or the like may also be a related factor, although no details have been clarified yet.

When the base loss and/or adduct detection easily occurs, the ionization of the nucleic acid molecule as the analysis target will produce not only the molecular-related ion ([M+H]⁺) but also a plurality of ion species and will be detected in the form of multiple separate peaks of different species, which will consequently cause a decrease in the sensitivity with which the molecular-related ion is detected. Furthermore, the detection of the ion peaks of the multiple species makes the data analysis more complex. Therefore, a matrix with which the base loss and adduct peak detection will be suppressed more is preferred.

Each mass spectrum in Fig. 1 is a mass spectrum to be ultimately obtained in the measurement by a raster scan (i.e., a mass spectrum obtained by accumulating all sets of mass spectrum data at each measurement point). The sensitivity, resolution, base elimination state, adduct detection state in the table are based on the mass spectrum data shown in Fig. 1.

In the present example, the base elimination state was evaluated by the ratio (%) of the signal-to-noise ratio (S/N) value of a representative base-loss ion peak resulting from the base elimination to the S/N value of the [M+H]⁺ peak (specific S/N values are shown in the parentheses in the table). The adduct detection state was evaluated by the ratio (%) of the S/N value of a representative adduct peak resulting from the base elimination to the S/N value of the [M+H]⁺ peak (specific S/N values are shown in the parentheses in the table).

The uniformity of the peak detection by the raster scan was evaluated by the ratios (%) of the number of measurement points at which the [M+H]⁺ peak was detected with a sensitivity of S/N>2, the number of measurement points at which the [M+H]⁺ peak was detected with a sensitivity of S/N>5, and the number of measurement points at which the [M+H]⁺ peak was detected with a sensitivity of S/N>10, to the total number of measurement points (in the present example, 25 points) in the raster scan (the specific numbers of measurement points are shown in the parentheses in the table).

As shown in Fig. 1, the molecular-related ion [M+H]⁺ of the RNA was detected with a high sensitivity, particularly when the mixed matrices (DHAP+THAP) in which DHAP and THAP were mixed at ratios from 30:1 to 10:1 were used. When DHAP was solely used, the measurement points at which [M+H]⁺ was detected with a sensitivity of, for example, S/N> 10 were approximately 60% of the measurement points at which the measurement by the raster scan was performed ((a) in Fig. 1). When THAP was solely used, the measurement points at which [M+H]⁺ was detected with a sensitivity of S/N>10 were approximately 20% of the measurement points at which the measurement by the raster scan was performed ((1) in Fig. 1). By comparison, when the mixed matrices (DHAP+THAP, with mixture ratios from 30:1 to 10:1) were used, the [M+H]⁺ peak was detected with a sensitivity of S/N>10 at equal to or more than 90% of the measurement points at which the measurement by the raster scan was performed ((b)-(e) in Fig. 1). The resolution was at roughly equal levels in all matrices. The base elimination state as well as the adduct peak detection state were suppressed to roughly equal levels in all matrices.

Thus, it was confirmed that the use of the mixed matrix (DHAP+THAP, with a mixture ratio from 30:1 to 10:1) improves the detection sensitivity of the molecular-related ion of the RNA as well as improves the uniformity of the measurement by a raster scan as compared to the conventional method in which DHAP or THAP is solely used.

### SECOND EXAMPLE

A mass spectrometric analysis of an RNA (sense strand of patisiran) was performed by a similar method to the first example except for <2. Preparation of Matrix Solutions>, in which the DHAP and THAP solutions were mixed together at each of the mixture ratios of 100:1, 50:1, 30:1, 20:1, 15:1, 10:1, 5:1 and 1:1 (v/v) to prepare mixed matrix solutions.

### <Result>

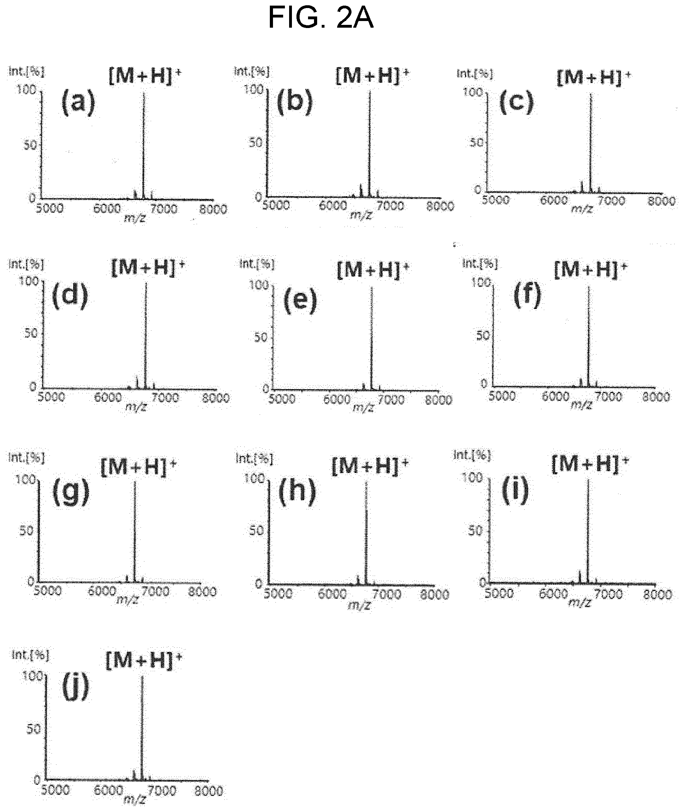

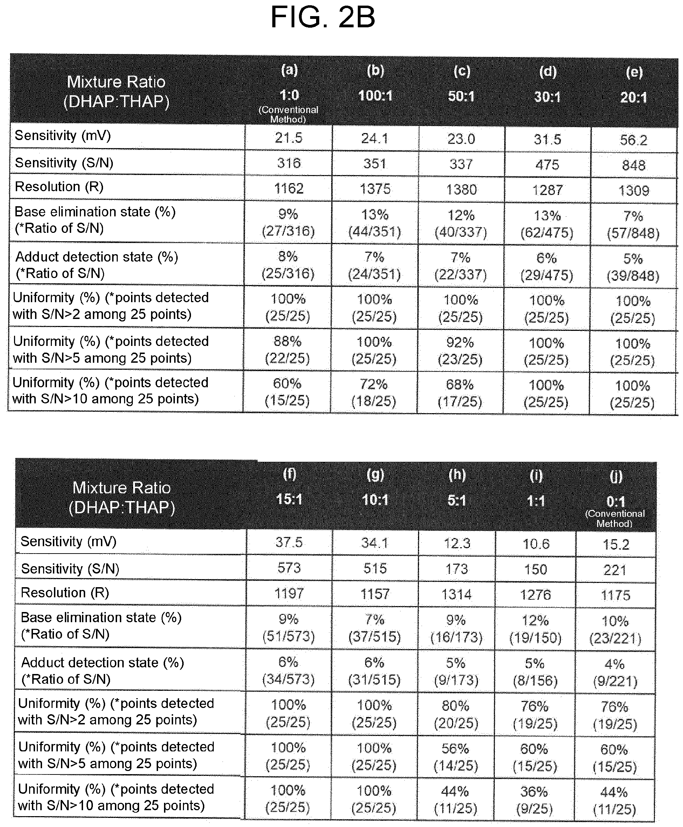

Fig. 2 shows mass spectra of the sense strand of patisiran as well as a table showing the state of the [M+H]⁺ peak detection (sensitivity, resolution, base elimination state, adduct detection state, and uniformity of the peak detection by the raster scan measurement) in the case where DHAP, THAP and mixed matrices (DHAP+THAP) which contained DHAP and THAP at the respective mixture ratios were used as matrices. The measurement of the second example was performed on a different day from the first example.

In Fig. 2, it was similarly confirmed that the use of the mixed matrix (DHAP+THAP, with a mixture ratio from 30:1 to 10:1) improves the sensitivity and uniformity as compared to the conventional method in which DHAP or THAP is solely used.

### THIRD EXAMPLE

A mass spectrometric analysis of an RNA (antisense strand of patisiran) was performed by a similar method to the first example except for <1. Preparation of Sample Solution> and <2. Preparation of Matrix Solutions>.

### <1. Preparation of Sample Solution>

As a sample solution, a 20-pmol/µL aqueous solution of patisiran (5'-A-U-G-G-A-A-Um-A-C-U-C-U-U-G-G-U-Um-A-C-dT-dT-3', where dT represents thymidine deoxyribonucleotide and Um represents 2'-O-methyluridine; Sequence Number 2) was prepared. (This sample was obtained through the desalting purification of a synthetic nucleic acid for research and development. Although patisiran is originally a double-stranded RNA consisting of the sense and antisense strands, only the antisense strand was used in the present example. The antisense strand had the entire length of 21 bases and a core sequence of 19 bases, with a DNA overhang of two bases (dTdT) at the 3' terminal; MW 6660).

### <2. Preparation of Matrix Solutions>

Similar to the first example, the DHAP and THAP solutions were prepared as matrix solutions. The prepared DHAP and THAP solutions were mixed together at each of the mixture ratios of 30:1 and 20:1 (v/v) to prepare mixed matrix solutions. Additionally, a 40mg/mL-50% acetonitrile-water solution of 3-hydroxypicolinic acid (3-HPA) containing 70 mM of diammonium hydrogen citrate as a matrix additive (3-HPA solution) was prepared as a matrix solution.

### <Result>

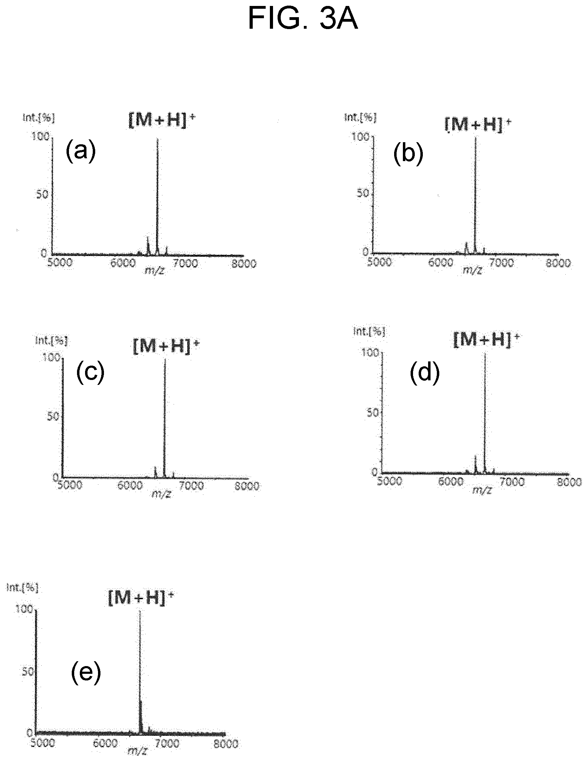

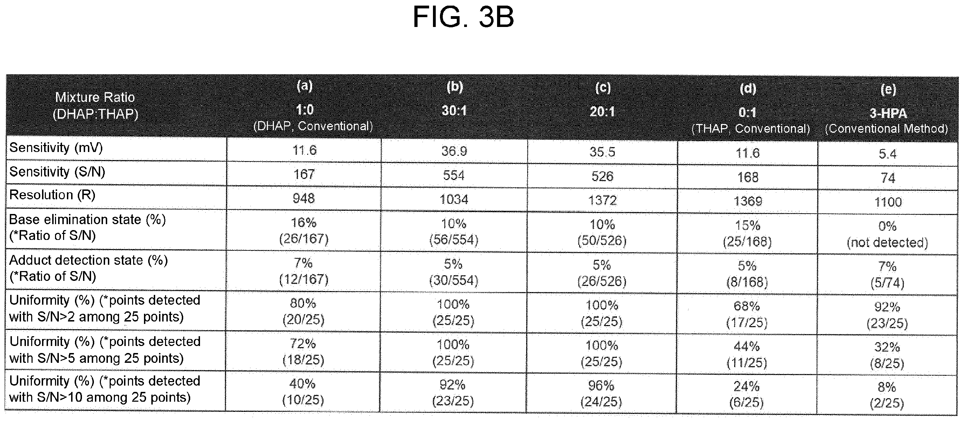

Fig. 3 shows mass spectra of the antisense strand of patisiran as well as a table showing the state of the [M+H]⁺ peak detection (sensitivity, resolution, base elimination state, adduct detection state, and uniformity of the peak detection by the raster scan measurement) in the case where DHAP, THAP, mixed matrices (DHAP+THAP) which contained DHAP and THAP at the respective mixture ratios, as well as 3-HPA were used as matrices.

As shown in Fig. 3, when the mixed matrices (DHAP+THAP, with mixture ratios of 30:1 and 20:1) were used, [M+H]⁺ was detected with high sensitivity as compared to the conventional method in which the DHAP, THAP or 3-HPA was solely used. Additionally, when the mixed matrices were used, the [M+H]⁺ peak was detected with a sensitivity of S/N> 10 at equal to or more than 90% of the measurement points at which the measurement by the raster scan was performed. Furthermore, when the mixed matrices were used, the base loss and adduct peak detection were suppressed to comparatively low levels, while the resolutuion was comparable to the levels achieved with the DHAP, THAP or 3-HPA matrix.

Thus, it was confirmed that the use of the mixed matrices (DHAP+THAP, with mixture ratios of 30:1 and 20:1) also enables the detection of [M+H]⁺ with high sensitivity and uniformity as well as sufficient resolution, base elimination state and adduct detection state for the antisense strand in addition to the sense strand of patisiran which was the RNA subjected to the measurement in the first example.

### FOURTH EXAMPLE

A mass spectrometric analysis of an RNA (sense strand of patisiran) was performed by a similar method to the first example except for <2. Preparation of Matrix Solutions>.

### <2. Preparation of Matrix Solutions>

The DHAP and THAP solutions were prepared as matrix solutions by a similar method to the first example. The prepared DHAP and THAP solutions were mixed together at each of the mixture ratios of 30:1 and 20:1 (v/v) to prepare mixed matrix solutions. Additionally, the 3-HPA solution was prepared by a similar method to the third example. The prepared 3-HPA and THAP solutions were mixed together at each of the mixture ratios of 1:1 and 1:3 (v/v) to prepare mixed matrix solutions.

### <Result>

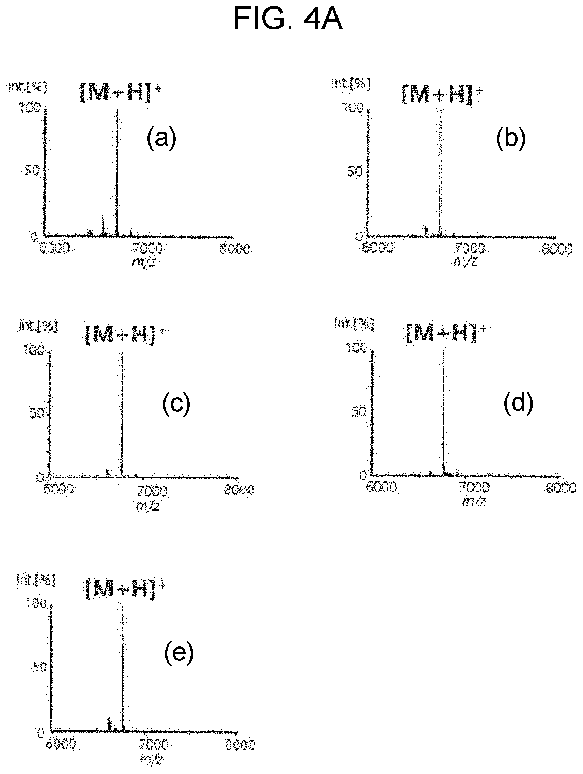

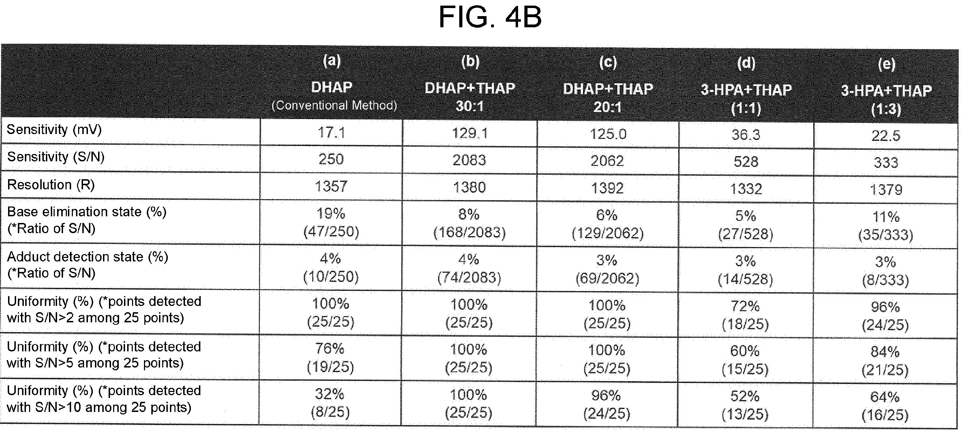

Fig. 4 shows mass spectra of the sense strand of patisiran as well as a table showing the state of the [M+H]⁺ peak detection (sensitivity, resolution, base elimination state, adduct detection state, and uniformity of the peak detection by the raster scan measurement) in the case where the DHAP, the mixed matrices (DHAP+THAP) which contained DHAP and THAP at the respective mixture ratios, as well as the mixed matrices (3-HPA+THAP) which contained 3-HPA and THAP at the respective mixture ratios were used as matrices.

As shown in Fig. 4, a comparison of DHAP+THAP and 3-HPA+THAP demonstrated that [M+H]⁺ was detected with higher sensitivity and uniformity when DHAP+THAP was used. As for the resolution, base elimination state and adduct detection state, the two mixed matrices were comparable to each other. Thus, it was confirmed that the combination of DHAP and THAP is particularly effective for improving the sensitivity and uniformity in RNA analysis.

### FIRST REFERENCE EXAMPLE

A mass spectrometric analysis of a DNA was performed by a similar method to the first example except for <1. Preparation of Sample Solution> and <2. Preparation of Matrix Solutions> .

### <1. Preparation of Sample Solution>

As a sample solution, a 20-pmol/µL aqueous solution of mipomersen (5'-MG-MC-MC-MU-MC-dA-dG-dT-dC-dT-dG-dC-dT-dT-dC-MG-MC-MA-MC-MC-3' where M represents 2'-O-(2-methoxyethyl)nucleoside and d represents 2'-deoxynucleoside; the C5 carbon of cytosine and uracil are replaced by the methyl group, while the phosphodiester bonds at all inter-nucleotide sections are replaced by the phosphorothioate bonds; Sequence Number 3) was prepared. (This sample was obtained through the desalting purification of a synthetic nucleic acid for research and development; DNA, with the entire length of 20 bases; MW 7177).

### <2. Preparation of Matrix Solutions>

The DHAP and THAP solutions were prepared as matrix solutions by a similar method to the first example. The prepared DHAP and THAP solutions were mixed together at each of the mixture ratios of 50:1, 30:1, 20:1, 10:1, 5:1, 1:1, 1:5, 1:10 and 1:30 (v/v) to prepare mixed matrix solutions.

### <Result>

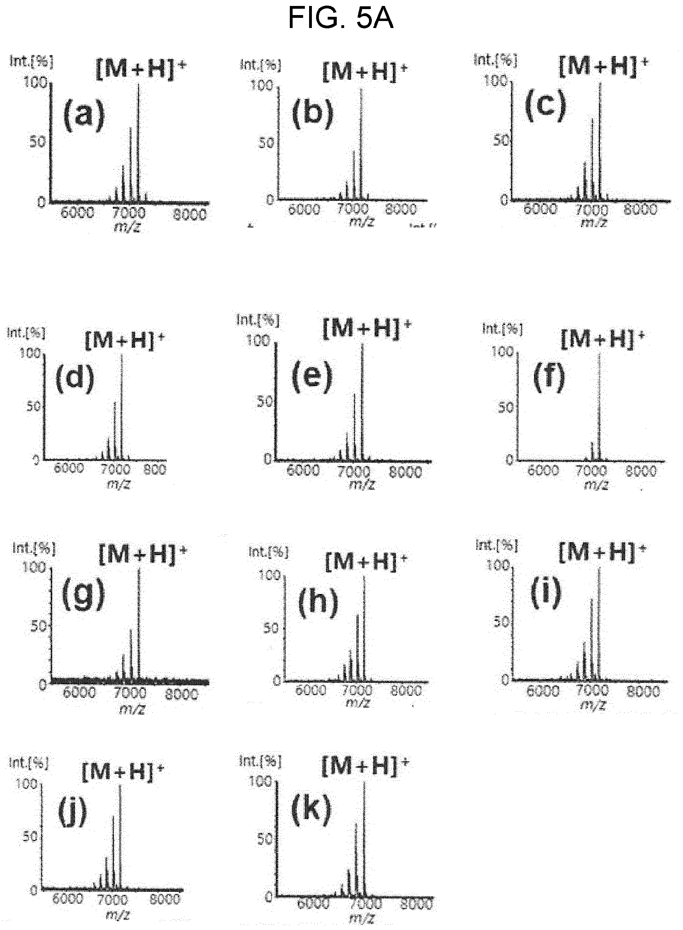

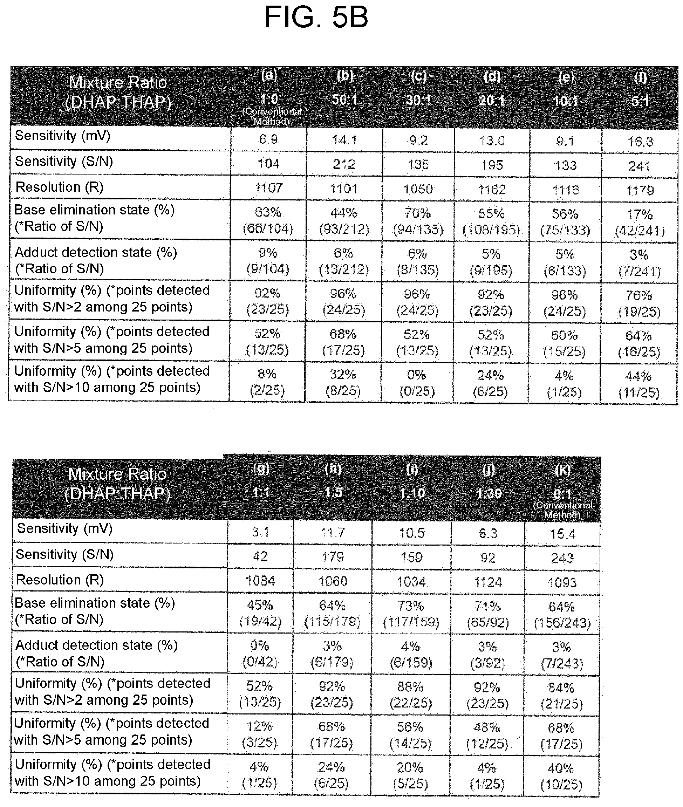

Fig. 5 shows mass spectra of mipomersen as well as a table showing the state of the [M+H]⁺ peak detection (sensitivity, resolution, base elimination state, adduct detection state, and uniformity of the peak detection by the raster scan measurement) in the case where DHAP, THAP and mixed matrices (DHAP+THAP) which contained DHAP and THAP at the respective mixture ratios were used as matrices.

As shown in Fig. 5, the effects of the sensitivity improvement and uniformity improvement by the mixed matrices (DHAP+THAP) were not recognized for mipomersen, which is a DNA. Thus, it was confirmed that DHAP+THAP is a matrix which is particularly effective in RNA analysis.

### [Modes]

It is evident for a person skilled in the art that the previously described illustrative embodiment is a specific example of the following modes of the present invention.

(Clause 1) A method for analyzing an RNA according to one mode of the present invention is a method in which a mixed matrix containing 2,4-dihydroxyacetophenone (DHAP) and 2,4,6-trihydroxyacetophenone monohydrate (THAP) is used as a matrix to analyze an RNA contained in a sample by a matrix-assisted laser desorption/ionization mass spectrometer.

The use of the mixed matrix containing 2,4-dihydroxyacetophenone and 2,4,6-trihydroxyacetophenone monohydrate improves the homogeneity of the sample/mixed-matrix mixture crystal to be formed as an analysis sample, consisting of an RNA-containing sample and the matrix, as well as the uniformity of the distribution of the RNA sample within the mixture crystal. Therefore, when an RNA contained in a sample is analyzed by means of a matrix-assisted laser desorption/ionization mass spectrometer, an appropriate amount of molecular-related ion of an analysis-target sample can be produced regardless of the laser irradiation position, so that the RNA can be satisfactorily analyzed in a comparatively uniform fashion, and therefore, easily and speedily. Additionally, the molecular-related ion of the RNA can be detected with a comparatively high sensitivity. Furthermore, the detection of the molecular-related ion can be achieved with a sufficient level of resolution while suppressing the base loss and adduct peak detection.

(Clause 2) In the method for analyzing an RNA according to Clause 1, the mixture ratio of DHAP and THAP in the mixed matrix may be within a range from 30:1 to 10:1.

This enables an analysis of an RNA with an even higher sensitivity. Furthermore, an appropriate amount of molecular-related ion of the RNA sample can be produced more uniformly. Therefore, the RNA can be satisfactorily analyzed easily and speedily.

(Clause 3) In the method for analyzing an RNA according to Clause 1, diammonium hydrogen citrate may be used as a matrix additive to the mixed matrix to analyze the RNA contained in the sample.

Diammonium hydrogen citrate particularly contributes to an improvement of the sensitivity for high-mass molecules. This enables an analysis of an RNA with an even higher sensitivity.

(Clause 4) The method for analyzing an RNA according to Clauses 1-3 may be a method in which a sample/mixed-matrix mixture solution is prepared by mixing a sample solution containing an RNA, a matrix solution containing DHAP and a matrix solution containing THAP, or a sample/mixed-matrix mixture solution is prepared by mixing a sample solution containing an RNA and a mixed matrix solution containing DHAP and THAP, and a sample/mixed matrix mixture prepared by dropping the sample/mixed-matrix mixture solution onto a sample plate and drying the same solution is subjected to a measurement by the matrix-assisted laser desorption/ionization mass spectrometer.

Preparing a sample/mixed-matrix mixture solution by previously mixing a sample solution containing an RNA, a matrix solution containing DHAP and a matrix solution containing THAP, or preparing a sample/mixed-matrix mixture solution by previously mixing a sample solution containing an RNA and a mixed matrix solution containing DHAP and THAP, further improves the homogeneity of the mixture crystal consisting of the RNA-containing sample and the mixed matrix (sample/mixed-matrix mixture) to be formed as an analysis sample on a sample plate, as well as the uniformity of the distribution of the RNA sample within the mixture crystal. Therefore, when an RNA contained in a sample is analyzed by means of a matrix-assisted laser desorption/ionization mass spectrometer, an appropriate amount of molecular-related ion of an RNA sample can be produced regardless of the laser irradiation position, so that the RNA can be satisfactorily analyzed in a comparatively uniform fashion, and therefore, easily and speedily, with an even higher sensitivity.

(Clause 5) The method for analyzing an RNA according to Clauses 1-3 may be a method in which a sample solution which contains an RNA, and a mixed matrix solution which contains DHAP and THAP, are separately dropped onto a sample plate to prepare a sample/mixed-matrix solution on the sample plate, and a sample/mixed-matrix mixture prepared by drying the sample/mixed-matrix solution is subjected to a measurement by the matrix-assisted laser desorption/ionization mass spectrometer.

By previously preparing the sample solution and the mixed matrix solution, and subsequently preparing the sample/mixed-matrix mixture, it becomes more likely that the matrices in the mixed matrix as well as the mixed matrix and the RNA will be uniformly mixed, so that the uniformity of the formation of the sample/mixed-matrix mixture as well as the uniformity of the distribution of the RNA sample within the same mixture will be further improved. Therefore, the RNA molecule can be detected in a comparatively uniform fashion, and therefore, easily and speedily, with an even higher sensitivity.

(Clause 6) The method for analyzing an RNA according to Clauses 1-3 may be a method in which a sample solution containing an RNA, a matrix solution containing DHAP and a matrix solution containing THAP are separately dropped onto a sample plate to prepare a sample/mixed-matrix solution on the sample plate, and a sample/mixed-matrix mixture prepared by drying the sample/mixed-matrix solution is subjected to a measurement by the matrix-assisted laser desorption/ionization mass spectrometer to analyze the RNA contained in the sample.

By previously preparing the sample solution and the matrix solutions, and subsequently preparing the sample/mixed-matrix mixture, it becomes more likely that the matrices in the mixed matrix as well as the mixed matrix and the RNA will be uniformly mixed, so that the homogeneity of the sample/mixed-matrix mixture as well as the uniformity of the distribution of the RNA sample within the same mixture will be further improved. Therefore, the RNA molecule can be detected in a comparatively uniform fashion, and therefore, easily and speedily, with an even higher sensitivity.

(Clause 7) In the method for analyzing an RNA according to Clauses 1-6, the matrix-assisted laser desorption/ionization mass spectrometer may be a digital ion trap type.

In a digital ion trap type of mass spectrometer, fragmentation is more likely to occur than in a time-of-flight type of device. Furthermore, its sensitivity and resolution are likely to be affected by the amount of ions. Accordingly, the effects of the present invention are more easily obtained, and the RNA can be more effectively analyzed.

(Clause 8) A matrix for matrix-assisted laser desorption/ionization mass spectrometry for RNA according to one mode of the present invention is a matrix containing 2,4-dihydroxyacetophenone (DHAP) and 2,4,6-trihydroxyacetophenone monohydrate (THAP).

When an RNA contained in a sample is analyzed by means of a matrix-assisted laser desorption/ionization mass spectrometer, the use of this matrix makes it possible to produce an appropriate amount of molecular-related ion of an RNA sample in a comparatively uniform fashion and detect it with a higher sensitivity, so that the RNA can be satisfactorily analyzed.

## Claims

1. A method for analyzing an RNA, wherein a mixed matrix containing 2,4-dihydroxyacetophenone (DHAP) and 2,4,6-trihydroxyacetophenone monohydrate (THAP) is used as a matrix to analyze an RNA contained in a sample by a matrix-assisted laser desorption/ionization mass spectrometer.

2. The method for analyzing an RNA according to claim 1, wherein a mixture ratio of DHAP and THAP in the mixed matrix is within a range from 30:1 to 10:1.

3. The method for analyzing an RNA according to claim 1, wherein diammonium hydrogen citrate is used as a matrix additive to the mixed matrix to analyze the RNA contained in the sample.

4. The method for analyzing an RNA according to one of claims 1-3, wherein a sample/mixed-matrix mixture solution is prepared by mixing a sample solution containing an RNA, a matrix solution containing DHAP and a matrix solution containing THAP, or a sample/mixed-matrix mixture solution is prepared by mixing a sample solution containing an RNA and a mixed matrix solution containing DHAP and THAP, and a sample/mixed matrix mixture prepared by dropping the sample/mixed-matrix mixture solution onto a sample plate and drying the same solution is subjected to a measurement by the matrix-assisted laser desorption/ionization mass spectrometer.

5. The method for analyzing an RNA according to one of claims 1-3, wherein a sample solution which contains an RNA, and a mixed matrix solution which contains DHAP and THAP, are separately dropped onto a sample plate to prepare a sample/mixed-matrix solution on the sample plate, and a sample/mixed-matrix mixture prepared by drying the sample/mixed-matrix solution is subjected to a measurement by the matrix-assisted laser desorption/ionization mass spectrometer.

6. The method for analyzing an RNA according to one of claims 1-3, wherein a sample solution containing an RNA, a matrix solution containing DHAP and a matrix solution containing THAP are separately dropped onto a sample plate to prepare a sample/mixed-matrix solution on the sample plate, and a sample/mixed-matrix mixture prepared by drying the sample/mixed-matrix solution is subjected to a measurement by the matrix-assisted laser desorption/ionization mass spectrometer.

7. The method for analyzing an RNA according to one of claims 1-3, wherein the matrix-assisted laser desorption/ionization mass spectrometer is a digital ion trap type.

8. A matrix for matrix-assisted laser desorption/ionization mass spectrometry for RNA, wherein the matrix contains 2,4-dihydroxyacetophenone (DHAP) and 2,4,6-trihydroxyacetophenone monohydrate (THAP).
